(19) Europäisches Patentamt
European Patent Office
Office européen des brevets

(11) EP 1 479 730 A1

(12) EUROPEAN PATENT APPLICATION

(43) Date of publication:
**24.11.2004 Bulletin 2004/48**

(51) Int Cl.[7]: **C09C 1/00**, C09C 1/30, C09C 1/24

(21) Application number: **04009621.6**

(22) Date of filing: **23.04.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **20.05.2003 EP 03011414**

(71) Applicant: **Merck Patent GmbH**
**64293 Darmstadt (DE)**

(72) Inventors:
- **Heider, Lilia, Dr.**
  **Winchester SO22 4HZ (GB)**
- **Riddle, Rodney, Dr.**
  **Poole Dorset BH12 3AG (GB)**
- **Weller, Mark T., Prof.**
  **Old Basing Basingstoke RG248WE Hants (GB)**
- **Armstrong, Jennifer**
  **S25 2QZ Sheffield South Yorkshire (GB)**

(54) **Pigments based on binary phosphate systems**

(57) The present invention relates to a novel effect pigment based on substrates comprising at least one layer, which consists of a binary phosphate system, the production of these pigments and their use.

EP 1 479 730 A1

## Description

The present invention relates to a novel effect pigment based on substrates comprising at least one layer, which consists of a binary phosphate system, the production of these pigments and their use.

Existing lustre effect pigments are based on metal oxide coatings on suitable substrates. This can include multiple layers of different oxides on various substrates including mica, silicon dioxide or aluminium oxide. These products have colours generated by interference effects and absorption properties of the metal oxides combined with a lustrous appearance. However, without addition of absorbing materials, such as inorganic absorbing metal oxides, carbon black or organic dyes, a limited range and depth of colours only is possible in these pigments.

JP 21456/1983 describes colour flake pigments based on non-metallic inorganic flake pigments coated with an outer layer of an insoluble metal phosphate as a binding vehicle containing an insoluble organic pigment. The metal of the insoluble metal phosphate can be selected from magnesium, calcium, zinc, aluminium, titanium or zirconium. Due to the transparency of the above-mentioned metal phosphates the absorption colour of the outer layer is based on the organic pigment. The coprecipitation of the organic pigment into a metal phosphate layer is disadvantageous in respect of a possible non-uniform distribution of the absorption pigment within the metal phosphate.

It is therefore an object of the present invention to provide new coloured effect pigments, which combine a lustre or brilliant appearance with great colour intensity and hue as well as a good tinting strength. The pigments should show good adhesion properties to be smooth to the touch.

Furthermore, the effect pigments should advantageously show a uniform colour appearance.

Surprisingly it has been found that effect pigments according to the present invention can fulfil these objectives in a more effective manner. Therefore, the present invention describes effect pigments based on substrates comprising at least one layer, which consists of a binary phosphate system.

Such objects are achieved by a process for preparing the pigment according to the present invention, in which a substrate is coated with an inorganic phosphate followed by annealing to form the binary phosphate system.

The invention additionally provides for the use of the pigments of the invention for pigmenting paints, lacquers, printing inks, powder coatings, paper coatings, plastics, cosmetics, inks, glazes for ceramics and glasses, decorative applications for foods and drugs and security-enhancing features. For this purpose they can also be employed as mixtures with commercially customary pigments, examples being organic and inorganic absorption pigments, effect pigments and LCP pigments, e.g. hiding white, coloured and black organic and inorganic pigments and also with conventional transparent, coloured and black lustre pigments based on metal oxide coated mica, $TiO_2$ flakes, $SiO_2$ flakes or $Al_2O_3$ flakes and coated or uncoated metal pigments, BiOCl pigments, platelet-shaped iron oxides or graphite flakes. The inventive pigments can be further coated with organic or inorganic layers to yield combination pigments.

Substrates that can be used in the present invention as base material on which the binary phosphate system is deposited include platelet-shaped, spherical or needle-shaped substrates. Preferably the substrates comprise but are not limited to micaceous iron oxide, natural (for example as in WO 99/48634), synthetic or doped (for example as in EP-A 0 068 311) micas (muscovite, phlogopite, fluoro-phlogopite, synthetic fluorophlogopite, talc, kaolin), basic lead carbonate, platelet-shaped barium sulphate, $SiO_2$-, $Al_2O_3$-, $TiO_2$-, Glass-, ZnO-, $ZrO_2$-, $SnO_2$-, BiOCl-, chromium oxide-, BN-, MgO-flakes, magnesium fluoride, $Si_3N_4$, graphite, effect pigments (for example EP-A 9 739 066, EP-A 0 948 571, WO 99/61529, EP-A 1 028 146, EP-A 0 763 573, US-A 5,858,078, WO 98/53012, WO 97/43348, US-A 6,165,260, DE-A 15 19 116, WO 97/46624, EP-A 0 509 352), effect multilayer pigments (for example EP-A 0 948 572, EP-A 0 882 099, US-A 5,958,125, US-A 6,139,613) and/or metals. Preferably, the metal is aluminium and/or titanium, most preferably passivated by inorganic treatment. Furthermore, coated $SiO_2$ spheres (for example EP-A 0 803 550, EP-A 1 063 265, JP-A 11 322 324), uncoated $SiO_2$ spheres (Ronaspheres® , all spheres described as starting materials in EP-A 0 803 550, EP-A 1 063 265, JP-A 11 322 324), micro bubbles (US-A 4,985,380), as well as needle-shaped metal oxides, preferably iron oxide, can be used as substrates to form a non-lustrous coloured pigment. In particular the substrate is of metal oxide coated mica, $Al_2O_3$, $SiO_2$, $TiO_2$ or glass. Especially preferred are multilayer pigments comprising alternating layers of metal oxide, metal oxide hydrate, metal and/or metal fluoride with a refractive index $n > 1.8$ and a metal oxide, metal oxide hydrate, metal and/or metal fluoride with a refractive index $n \leq 1.8$. These multilayer effect pigments are characterised through an intensively lustrous appearance and an angle-dependent interference colour.

The size of the substrates is not critical. Preferably, the mean diameter in the case of for example the platelet-shaped substrates can vary between 5 and 200 µm, preferably between 10 and 150 µm. The mean diameter of the spherical particles can vary between 10 nm and 100 µm, preferably between 500 nm and 50 µm and most preferably from 1 to 20 µm.

Effect pigments according to the present invention comprise at least one layer of a binary phosphate system. For example, the phosphate system can be a poly- or pyrophosphate structure.

In particular, the binary phosphate system has the formula

$$A_xP_yO_z$$

with

A = elements of group 1 to 14 of the periodic table, lanthanides or actinides
x = 1, 2....n
y = 1, 2,....m, defined through the poly- or pyrophosphate structure
z is defined through the phosphorus valence and the valence of the A-cation

Preferably, the binary phosphate system may consist of $MnPO_4$, $Cu_4O(PO_4)_2$, $AlPO_4$, $FePO_4$, $CePO_4$, $YPO_4$, $LaPO_4$, $BiPO_4$, $Mh(PO_3)_3$, $Ti(PO_3)_3$, $Zn_3(PO_4)_2$, $Ca_3(PO_4)_2$, $Ti_3(PO_4)_4$, Diphosphates of M = $M_4(P_2O_7)_3$ with M = e.g. V, Cr, Fe and especially preferred are $Cu_4O(PO_4)_2$, $Cu_3(PO_4)_2$, $FePO_4$, $BiPO_4$ and $Mn(PO_3)_3$.

The thickness of the layer of the binary phosphate system can vary between 10 and 300 nm, preferably between 20 and 200 nm depending on the required colour effect of the pigments.

For the preparation of the binary phosphate system a substrate as described above is coated with an inorganic phosphate, followed by annealing to form the binary phosphate phase composition.

The deposition of the inorganic phosphate is performed wet chemically using appropriate precursors. The inorganic phosphate can be deposited directly by precipitation using a solution of the constituent of the binary phosphate system and a phosphate source. The phosphate source can be any inorganic phosphate, such as primary, secondary or tertiary alkaline or earth alkaline metal phosphates, e.g. Sodium phosphate, Disodium hydrogenphosphate, Sodium dihydrogenphosphate, Ammonium dihydrogenphosphate, Diammonium hydrogenphosphate, Pyrophosphates, Polyphosphates or Phosphoric acid. Especially preferred are Disodiumhydrogenphosphate, Diammonium hydrogenphosphate, Pyrophosphates, Polyphosphates and Phosphoric acid. Process parameters such as pH-value or temperature of the solution are controlled to obtain optimal conditions for precipitation.

For the precipitation of the constituents of the binary phosphate system in the deposition process all known soluble compounds of these constituents may be used as source, such as halides, nitrates, oxalates or other organic acid salts and/or sulphates, preferably the chlorides and/or nitrates. As well as in the case of the phosphates the deposition parameters can be optimised.

After deposition the resulting product can be filtrated and dried prior to annealing. The drying can be performed at temperatures in the range of 80 and 120°C, preferably between 100 and 120°C. The period of drying can vary between 6 and 24 hours, preferably between 8 and 14 hours. Any person skilled in the art can perform the control and optimization of the process parameters.

To transform the above described deposited layer into the binary phosphate phase an annealing is performed. The temperature for annealing can vary between 500 and 900°C, preferably between 600 and 850°C. Annealing can be performed under oxidising, reducing and/or inert atmospheres, depending on the desired binary phosphate system. Oxidising atmospheres contain oxygen, air and/or mixtures thereof. Hydrogen alone or in mixture with nitrogen and/or argon may be used as reducing atmosphere. Pure nitrogen, pure argon and/or mixtures thereof are suited for usage as inert atmospheres. Any person skilled in the art can perform the control and optimization of the process parameters.

Preferred embodiments for the process for the formation of an inorganic phosphate are as follows:

$$\text{Mica} + 4\,CuCl_2 + 2\,(NH_4)_2HPO_4 \xrightarrow{NaOH} \text{Mica} + Cu_4O(PO_4)_2 + 4\,NH_4Cl + 2\,HCl + 2\,NaCl$$

$$\text{Mica} + FeCl_3 + Na_2HPO_4 \xrightarrow{NaOH} \text{Mica} + FePO_4 + 2\,NaCl + HCl$$

$$\text{Mica} + Bi(NO_3)_3 + H_3PO_4 \xrightarrow{NaOH} \text{Mica} + BiPO_4 + 3\,HNO_3$$

$$\text{Mica} + Bi(NO_3)_3 + (NH_4)_2HPO_4 \xrightarrow{NaOH} \text{Mica} + BiPO_4 + 2\,(NH_4)NO_3 + HNO_3$$

$$Mica + 3\,ZnCl_2 + 2\,Na_2HPO_4 \xrightarrow{NaOH} Mica + Zn_3(PO_4)_2 + 4\,NaCl + 2\,HCl$$

The new coloured effect pigments can be used as substrate to precipitate further optical layers. If desired, the pigments according to the present invention can be further coated with one or more layers of metal oxides, metal oxide hydrates, metal fluorides and/or semitransparent metal layers on top of the binary phosphate system. The binary phosphate layer can also be placed as an intermediate layer of metal oxide, metal oxide hydrate, metal fluoride and/or semitransparent metal stacks. The metal oxide can be selected from any metal oxide, preferably from titanium oxide, iron oxide, aluminium oxide, aluminium oxide hydrate, silicon oxide, silicon oxide hydrate, zirconium oxide, chromium oxide, zinc oxide, tin oxide, antimony oxide, indium oxide, potassium ferric ferro cyanides, most preferably from titanium oxide, iron oxide, aluminium oxide, aluminium oxide hydrate, silicon oxide, silicon oxide hydrate and/or mixtures thereof. The metal fluoride is preferably magnesium fluoride. The metal of the semitransparent metal layer can be selected from chromium, molybdenum, aluminium, silver, platinum, nickel, copper and/or gold, preferably from aluminium, silver. In particular, the metal oxide, metal oxide hydrate, metal fluoride and/or semitransparent metal layers are arranged as alternating layers of metal oxide, metal oxide hydrate, metal and/or metal fluoride with a refractive index $n > 1.8$ and a metal oxide, metal oxide hydrate, metal and/or metal fluoride with a refractive index $n \leq 1.8$. Pigments according to this embodiment combine the colour of the binary phosphate system with an intensively lustrous appearance and may show an angle-dependent interference colour.

Preferred examples for metal oxides, metal oxide hydrates and/or metals with a refractive index $n > 1.8$ are titanium oxide, iron oxide, iron titanate, iron, chromium, silver and/or nickel, preferably titanium oxide, iron oxide, iron titanate.

Preferred examples for metal oxides, metal oxide hydrates, metals and/or metal fluorides with a refractive index $n \leq 1.8$ are silicon oxide, silicon oxide hydrate, aluminium oxide, aluminium oxide hydrate, aluminium and/or magnesium fluoride.

Examples for the constitution of pigments according to the present invention are given in the following:

$$\textit{Substrate} + Cu_4O(PO_4)_2 \rightarrow \textit{substrate plus binary phosphate system}$$

$$\textit{Substrate} + BiPO_4 \rightarrow \textit{substrate plus binary phosphate system}$$

$$\textit{Substrate} + TiO_2 + BiPO_4 \rightarrow \textit{metal oxide coated substrate plus binary phosphate system}$$

$$\textit{Substrate} + TiO_2 + SiO_2 + TiO_2 + BiPO_4$$

$$\rightarrow \textit{multilayer coated substrate plus binary phosphate system}$$

$$\textit{Substrate} + BiPO_4 + TiO_2 + SiO_2 + TiO_2$$

Furthermore, the resulting pigments can be coated with inorganic and/or organic compounds to increase their weather stability respectively their photo stability. Useful methods are for instance described in US-A 4,134,776, EP-A 0 649 886, WO 97/29059 and references cited therein.

Pigments according to the present invention show a high value of chroma, good tinting strength, and pure colour and in the case of platelet-shaped substrates an interesting lustre. They offer the generation of new colours that cannot be produced by using systems of the state of the art like $TiO_2$, iron oxides or organic pigments coated substrates. Furthermore, these effects cannot be achieved through physical mixtures of interference colour pigments and absorbing systems. Depending on the type and thickness of the substrate as well as on the hue and colour intensity of the binary phosphate system many different optical effects are possible. Transparent platelet-shaped substrates such as mica offer the creation of lustrous coloured pigments. Coatings on metallic, semi-transparent or coloured substrates provide overlapping colour effects of absorption, reflection and interference combined with a greater hiding strength. For example, pigments comprising one or more $BiPO_4$ layers show a silver white colour combined with a soft lustrous appearance. In the case of spherical substrates pigments with a great variety in colour are obtainable. Due to their optical behaviour interesting absorption colours without lustre can be observed.
In addition and in contrast to pigments comprising organic pigment containing layers, the new effect pigments show a uniform colour.

Furthermore, pigments according to the present invention show a good dispersibility in all application media. The combination of new colour effects combined with good application behaviour in pigments according to the present invention is useful in a wide range of applications such as paints, lacquers, printing inks, powder coatings, paper coatings, plastics, cosmetics, inks, glazes for ceramics and glasses, decorative applications for foods and drugs and security-enhancing features.

The pigments and their production process according to the present invention is more illustratively demonstrated but not limited by means of the following examples.

**Examples:**

Example 1: Realisation of $BiPO_4$ onto mica

100 g of mica are suspended in 1.9 litres of fully deionised water. The suspension is heated to 75°C. The pH of the solution is adjusted to pH 1-5, preferable at 4 by addition of nitric acid or hydrochloric acid. To realise a 50 g $BiPO_4$ coating onto mica roughly 276.5 g of a bismuth nitrate solution (12.4 % Bismuth content) are slowly added to the reactor. The pH-value is maintained via a relevant amount of Di-sodium hydrogen-phosphate solution (e.g. 0.8 % Phosphate content) or a relevant amount (107.1 g) of a diluted phosphoric acid solution (e.g. 15% Phosphate content) to form $BiPO_4$ and NaOH (32% NaOH) in addition. The suspension is equilibrated for 30 min after final solution adding.

The suspension is cooled down and the solid remaining is decanted and washed to remove all side products. The pigment is transferred to a porcelain crucible and dried over night at 110°C. After sieving with a 0.3 mm sieve the powder is calcined at 700°C for 30 min. A silver $BiPO_4$ pigment with lustre is obtained.

L value 93-94, a = 0.5-1, b = 2-4
These values are measured using a Minolta colour unit CR-300 Nr. The Lab values can differ depending on the applied layer thickness and morphology of the layer.

Example 2: Realisation of $Cu_4O(PO_4)_2$ onto mica

100 g of mica are suspended in 1.9 litres of fully deionised water. The suspension is heated to 75°C. The pH of the solution is adjusted to pH 3-7 by addition of nitric acid or hydrochloric acid. 390.95 g of a copper (II) chloride solution (15% Copper content) are slowly added to the reactor. The pH-value is maintained via Disodium hydrogen-phosphate or phosphoric acid solution taking the final stoichiometry into account (142.43 g of a e.g. 15% solution). The suspension is equilibrated for 30 min. The suspension is cooled down and the solid remaining is decanted and washed to remove all side products. The pigment is transferred to a porcelain crucible and dried over night at 110°C. After sieving with a 0.3 mm sieve the powder is calcined at 880°C for 30 min. A green $Cu_4O(PO_4)_2$ pigment with lustre is obtained.
L value = 50-57, a = -13 - -25, b = 25-35, C value = 30-37, h° value = 125-135
These values are measured using a Minolta colour unit CR-300. The Lab values can differ depending on the applied layer thickness and morphology.

Example 3: Eye Shadow

| Phase A | |
|---|---|
| 30% | Pigment of Example 1 |
| 7.5% | Potato starch |
| 49.5% | Talc |
| 2.5% | Magnesium stearate |
| Phase B: | |
| 9.14% | Isopropyl stearate |
| 0.53% | Cetyl palmitat |

(continued)

| Phase B: | |
|---|---|
| 0.53% | Ewalin 1751 (Petrolatum) |
| 0.2% | Fragrance Elegance 79228 D MF (Perfume) |
| 0.1 % | Propyl-4-hydroxybenzoate (Propylparabene) |
| Total: | 100% |

The components of Phase A are mixed homogeneously. Thereafter one adds the powder mixture with ongoing stirring to Phase B. The final powder will be pressed, using 40-50 bar pressure, afterwards.

Example 4: Nail varnish

2% Pigment of Example 2
98% Thixotrope nail varnish base 1348 (toluene, ethyl acetate, butyl acetate, nitrocellulose, tosylamide/formaldehyde resin, dibutyl phthalate, isopropyl alcohol, stearalkonium hectorite, camphor, acrylates copolymers, benzophenone)

All components are intensively mixed to get a homogeneous system.

## Claims

1. Effect pigments based on substrates comprising at least one layer, which consists of a binary phosphate system.

2. Effect pigments according to claim 1, **characterized in that** the binary phosphate system has the formula $A_xP_yO_z$ with

   A = elements of group 1 to 14 of the periodic table, lanthanides or actinides
   x = 1, 2....n
   y = 1, 2,....m, defined through the poly- or pyrophosphate structure
   z is defined through the phosphorus valence and the valence of the A-cation

3. Effect pigments according to claim 1 or 2, wherein the substrate is platelet-shaped, spherical or needle-shaped.

4. Effect pigments according to claim 3, wherein the substrate is platelet-shaped and is of mica, $SiO_2$, aluminium oxide, glass, micaceous iron oxide, oxidised graphite, aluminium oxide-coated graphite, basic lead carbonate, barium sulphate, chromium oxide, BN, MgO, magnesium fluoride, $Si_3N_4$, metal, effect pigments or effect multilayer pigments, or of coated or uncoated $SiO_2$-spheres or needle-shaped iron oxides.

5. Effect pigments according to claim 3, **characterised in that** the metal is aluminium or titanium.

6. Effect pigments according to one of claims 1 to 5, **characterised in that** the pigments are further coated on top of the binary phosphate system with one or more layers of metal oxides, metal oxide hydrates, metal fluorides and/or semitransparent metal layers.

7. Process for the preparation of a pigment according to claim 1, **characterised in that** a substrate is coated with an inorganic phosphate, followed by annealing to form the binary phosphate phase composition.

8. Process according to claim 7, **characterised in that** the substrate is platelet-shaped and is of mica, $SiO_2$, aluminium oxide, glass, micaceous iron oxide, oxidised graphite, aluminium oxide-coated graphite, basic lead carbonate, barium sulphate, chromium oxide, BN, MgO, magnesium fluoride, $Si_3N_4$, metal, effect pigments or effect multilayer pigments, or of coated or un-coated $SiO_2$-spheres or needle-shaped iron oxides.

9. Use of a pigment according to one of claims 1 to 6 in paints, lacquers, printing inks, powder coatings, paper

coatings, plastics, cosmetics, inks, glazes for ceramics and glasses, decorative applications for foods and drugs and security-enhancing features.

European Patent Office

# EUROPEAN SEARCH REPORT

Application Number
EP 04 00 9621

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 757 085 A (MERCK PATENT GMBH) 5 February 1997 (1997-02-05) * column 1, line 5 - line 12 * * column 4, line 45 - line 53 * * examples 1,3,6,7,9,10 * * claims * ----- | 1-5,7-9 | C09C1/00 C09C1/30 C09C1/24 |
| X | DATABASE WPI Section Ch, Week 200270 Derwent Publications Ltd., London, GB; Class E32, AN 2002-647193 XP002297771 & JP 2002 138218 A (ISHIHARA SANGYO KAISHA LTD) 14 May 2002 (2002-05-14) * abstract * ----- | 1-6,9 | |
| X | US 4 456 486 A (BERNHARD HORST) 26 June 1984 (1984-06-26) * column 1, line 51 - line 57 * * column 3, line 10 - line 13 * * column 3, line 61 - column 4, line 11 * * column 4, line 53 - column 5, line 10 * * examples 2,3 * ----- | 1-4,7-9 | TECHNICAL FIELDS SEARCHED (Int.Cl.7) C09C |
| X | EP 0 522 678 A (COOKSON LAMINOX LTD) 13 January 1993 (1993-01-13) * column 1, line 1 - line 13 * * column 1, line 47 - column 2, line 17 * ----- | 1-5,9 | |
| X | EP 0 753 546 A (TIOXIDE GROUP SERVICES LTD) 15 January 1997 (1997-01-15) * page 1, line 13 - line 14 * * page 1, line 26 - line 37; claims; examples * ----- | 1-5,9 | |

The present search report has been drawn up for all claims

| Place of search | Date of completion of the search | Examiner |
|---|---|---|
| Munich | 24 September 2004 | Nobis, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 00 9621

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2004

| Patent document cited in search report | Publication date | Patent family member(s) | Publication date |
|---|---|---|---|
| EP 0757085 A | 05-02-1997 | JP 9048930 A | 18-02-1997 |
| | | CN 1146470 A ,B | 02-04-1997 |
| | | DE 69625650 D1 | 13-02-2003 |
| | | DE 69625650 T2 | 13-11-2003 |
| | | EP 0757085 A2 | 05-02-1997 |
| | | TW 396194 B | 01-07-2000 |
| | | US 5688314 A | 18-11-1997 |
| JP 2002138218 A | 14-05-2002 | NONE | |
| US 4456486 A | 26-06-1984 | DE 3137808 A1 | 31-03-1983 |
| | | BR 8205542 A | 30-08-1983 |
| | | CA 1184003 A1 | 19-03-1985 |
| | | CS 233732 B2 | 14-03-1985 |
| | | EP 0075755 A2 | 06-04-1983 |
| | | ES 8306497 A1 | 01-09-1983 |
| | | IN 157834 A1 | 05-07-1986 |
| | | JP 58069258 A | 25-04-1983 |
| | | ZA 8206930 A | 30-11-1983 |
| EP 0522678 A | 13-01-1993 | EP 0522678 A2 | 13-01-1993 |
| EP 0753546 A | 15-01-1997 | AU 709309 B2 | 26-08-1999 |
| | | AU 5610196 A | 23-01-1997 |
| | | CA 2180688 A1 | 14-01-1997 |
| | | EP 0753546 A2 | 15-01-1997 |
| | | GB 2303366 A ,B | 19-02-1997 |
| | | GB 2333100 A ,B | 14-07-1999 |
| | | GB 2333101 A ,B | 14-07-1999 |
| | | JP 9031359 A | 04-02-1997 |
| | | NO 962896 A | 14-01-1997 |
| | | US 5785748 A | 28-07-1998 |

EPO FORM P0459

For more details about this annex : see Official Journal of the European Patent Office, No. 12/82